(19) Europäisches Patentamt
European Patent Office
Office européen des brevets

(11) **EP 2 805 682 B1**

(12) **EUROPÄISCHE PATENTSCHRIFT**

(45) Veröffentlichungstag und Bekanntmachung des Hinweises auf die Patenterteilung:
**20.03.2019 Patentblatt 2019/12**

(51) Int Cl.:
**A61B 18/12** *(2006.01)*

(21) Anmeldenummer: **13169105.7**

(22) Anmeldetag: **24.05.2013**

(54) **KOAGULATIONSEINRICHTUNG MIT ENERGIESTEUERUNG**

POWER CONTROLLED COAGULATION DEVICE

DISPOSITIF DE COAGULATION AVEC COMMANDE D'ÉNERGIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.11.2014 Patentblatt 2014/48**

(73) Patentinhaber: **Erbe Elektromedizin GmbH**
**72072 Tübingen (DE)**

(72) Erfinder:
• **Schall, Heiko**
**72622 Nürtingen (DE)**
• **Schäller, Daniel**
**72070 Tübingen (DE)**

(74) Vertreter: **Rüger Abel**
**Patentanwälte**
**Webergasse 3**
**73728 Esslingen (DE)**

(56) Entgegenhaltungen:
**EP-A1- 1 862 137      EP-A1- 2 499 982**
**EP-A2- 0 717 967      WO-A2-2008/102154**
**US-A1- 2013 006 237**

EP 2 805 682 B1

**Beschreibung**

Die Erfindung betrifft eine Einrichtung zur Gewebekoagulation, insbesondere zur Gewebefusion.

Es sind verschiedene elektrochirurgische Verfahren in Gebrauch, deren Effekt auf einer kontrollierten Denaturierung biologischen Gewebes beruht.

Beispielsweise offenbart die EP 1 862 137 A1 eine Koagulationseinrichtung mit einem Generator, der zwei Elektroden speist, zwischen denen biologisches Gewebe gefasst ist. Das Gewebe durchläuft während der Koagulation eine erste Phase I, während derer die Gewebeimpedanz deutlich abnimmt und eine zweite Phase II, während derer die Gewebeimpedanz wieder ansteigt. Zur Erfassung der Gewebeimpedanz ist ein Sensorschaltkreis vorgesehen, der ein Abfragesignal aussendet, um die anfängliche Gewebeimpedanz zu bestimmen und nachfolgend eine bestimmte Trajektorie für den gewünschten Zeitverlauf der Gewebeimpedanz zu definieren. Das Abfragesignal wird durch einen elektrischen Puls gebildet, mit dem eine Gewebeeigenschaft gemessen wird. Die gemessene Gewebeeigenschaft kann Energie, Leistung, Impedanz, Strom, Spannung, elektrischer Phasenwinkel, reflektierte Leistung oder Temperatur sein.

Auch die US 8216223 B2 befasst sich mit der Koagulation von Gewebe. Während einer HF-Aktivierung von Elektroden wird zunächst die Gewebeimpedanz gemessen. Im weiteren zeitlichen Verlauf wird das Minimum der Impedanz festgestellt. Von diesem Punkt ausgehend wird eine Sollwertkurve für den gewünschten Impedanzanstieg generiert und ein Zielwert für die Impedanz errechnet. Wird dieser erreicht, wird der HF-Generator abgeschaltet. An das Abschalten schließt sich eine Kühlungsphase an, deren Länge ebenfalls aus der Sollwertkurve vorgegeben wird. Mit Ende der Kühlungsphase ist die Fusion abgeschlossen.

Ebenfalls anhand der initialen Gewebeimpedanz wird die Thermofusion gemäß US 8,034,049 B2 gesteuert. In der Phase I der Thermofusion wird zum Beispiel bei konstant gehaltenem Strom der Impedanzverlauf gemessen. Daraus werden die Initialimpedanz, der Abfall der Impedanz, das Minimum der Impedanz oder der Anstieg der Impedanz abgeleitet. Aus diesen Informationen werden andere Aktivierungsparameter generiert.

Die EP 2 213 255 B1 beschreibt die Steuerung der Energie bei einer Thermofusion. Dazu wird eine Zustandsvariable SV definiert, die das Fallen oder Steigen der Impedanz anzeigt. Es ist eine Sollwerttrajektorie für die Impedanz vorgegeben. Der Energieeintrag wird so gesteuert, dass der gewünschte zeitliche Impedanzverlauf angenähert wird. Dazu wird der Energieeintrag abhängig von der Zustandsvariablen SV zur Impedanz mitgekoppelt oder gegengekoppelt.

Die EP 2 394 593 A1 beschreibt die Messung der Impedanz während der Thermofusion. Es ist vorgesehen, nach Ablauf einer gewissen Mindestzeit zu überprüfen, ob eine Mindestimpedanz erreicht wurde. Sobald dies der Fall ist, wird die Aktivierung abgeschlossen.

Die US 6,733,498 B2 offenbart ein Verfahren zur Thermofusion, bei dem der zeitliche Verlauf der Gewebeimpedanz während der Applikation von HF-Spannung erfasst wird. Das Ende der ersten Phase und die Dauer der zweiten Phase werden durch den Impedanzverlauf entsprechend festgelegt.

Auch die US 8,147,485 B2 nutzt zur Regulierung der Thermofusion die Überwachung der Gewebeimpedanz. Aus dem Minimum der Gewebeimpedanz und dem Impedanzanstieg wird eine Impedanztrajektorie berechnet.

Auch die US 2010/0179563 A1 und die US 2011/0160725 A1 erfassen die Gewebeimpedanz oder deren Veränderung zur Steuerung oder Regelung des elektrochirurgischen Prozesses.

Die US 2013/006237 A1 beschreibt ein Koagulationsverfahren, bei dem in einer ersten Phase (Phase I) die in das Gewebe eingebrachte Energie gemessen und beschränkt wird, um das Koagulationsvolumen zu begrenzen. Sobald die festgelegte Energie in das Gewebe eingebracht worden ist, wird der Koagulationsprozess beendet.

Aus der EP 2 499 982 A1 ist es bekannt, einen Ablationsprozess zu steuern, wobei der Steueralgorithmus sowohl die Energie als auch die verstrichene Zeit als Rechengrößen berücksichtigt.

Die WO 2008/102154 A1 offenbart ein Verfahren zur Fusion einer Arterie unter Koagulation des in der Arterie enthaltenen Bluts. Dabei wird die Gewebeimpedanz überwacht und das Koagulationsende durch die Art der Impedanzänderung erfasst. Bei einer automatisierten Variante ist eine Leistungsregelschleife vorgesehen, wobei die Leistung oder andere Parameter der applizierten Spannung entweder nach einem vorberstimmten Muster oder in Abhängigkeit von der gemessenen Impedanz gesteuert werden können. Es wird außerdem vorgeschlagen, dass die Leistungsregelschleife die insgesamt applizierte Energie erfasst und überwacht

Der lokale Zustand von Gewebe wird durch die lokale spezifische Gewebeimpedanz charakterisiert. Die Erfassung der Impedanz zwischen zwei Elektroden liefert zwar einen Anhaltspunkt für den Zustand und somit den Behandlungsfortschritt des Gewebes insgesamt, wobei jedoch die lokale spezifische Gewebeimpedanz nicht erfasst wird. Dies kann zu Fehlschlüssen bei inhomogenem Gewebe führen.

Es ist Aufgabe der Erfindung, eine alternative Einrichtung zur Gewebekoagulation zu schaffen.

Diese Aufgabe wird mit der Einrichtung nach Anspruch 1 gelöst:

Die erfindungsgemäße Einrichtung dient zur Gewebekoagulation sowie bedarfsweise auch zur Gewebefusion. Dazu ist eine elektrische Quelle mit Elektroden zur Stromeinwirkung auf biologisches Gewebe verbunden oder verbindbar. Die elektrische Quelle kann eine Quelle für Gleichstrom oder Wechselstrom, vorzugsweise HF-Strom, sein. Die Quelle ist vorzugsweise steuerbar ausgebildet, um die Größe des abgegebenen Stroms und/oder der abgegebenen Spannung steuern zu können. Sie ist dazu an eine Steuereinheit angeschlossen. Diese enthält eine Überwachungseinheit, die mit der Quelle verbunden ist. Insbesondere ist die Überwachungseinheit mit dem Ausgang der Quelle verbunden, an den auch die Elektroden angeschlossen sind. Alternativ kann die Überwachungseinheit mit den Elektroden verbunden sein. Die Überwachungseinheit erfasst somit mindestens eine elektrische Größe, die die Energie kennzeichnet, die während einer ersten Betriebsphase von der Quelle an die Elektroden und somit von den Elektroden an das Gewebe abgegeben worden ist. Die erste Betriebsphase entspricht der Phase I der Gewebekoagulation, während derer der Gewebewiderstand abnimmt und ein Minimum durchläuft.

Zum Beispiel kann die Überwachungseinheit die aktuelle Leistung erfassen und diese während der ersten Betriebsphase aufintegrieren, um die abgegebene Energie zu ermitteln. Insbesondere ist es vorteilhaft, wenn die Überwachungseinheit die von den Elektroden abgegebene Wirkleistung erfasst. Durch Aufintegrieren wird daraus die Wirkenergie ermittelt, die im Gewebe thermisch umgesetzt worden ist. Die in der ersten Betriebsphase in das Gewebe eingebrachte Energie wird zur Steuerung der zweiten Betriebsphase genutzt. Diese entspricht der Phase II der Gewebekoagulation, während derer der Gewebewiderstand zunimmt und das Gewebe durch verkochen von Gewebeflüssigkeit trocknet.

Alternativ kann die Scheinleistung erfasst werden, die jedoch Blindleistungsanteile enthält. Sind diese bekannt oder konstant, kann auch die Scheinleistung und damit die insgesamt abgegebene Scheinenergie zur Steuerung der zweiten Betriebsphase genutzt werden.

Die Steuereinheit steuert die Quelle in der zweiten Betriebsphase anhand der während der ersten Betriebsphase ermittelten Energie (Wirkenergie oder Scheinenergie). Dadurch wird sichergestellt, dass die in der zweiten Betriebsphase applizierte Energiemenge an die Größe des von den Elektroden erfassten und beeinflussten Gewebeareals angepasst ist. Die in der ersten Phase I geöffneten Zellen setzen Gewebeflüssigkeit frei. In der zweiten Phase II wird diese unter Auftrocknung des Gewebes verdampft. Durch Erfassung der in der ersten Betriebsphase applizierten Energie steht ein Parameter zur Verfügung, anhand dessen die Phase II so gesteuert werden kann, dass das gesamte in Phase I elektrochirurgisch beeinflusste Gewebe gleichmäßig koaguliert wird.

Es ist zweckmäßig, wenn die Steuereinheit die Quelle in der ersten Betriebsphase I mit geregeltem Strom betreibt. Dabei ist es sowohl möglich, zu Beginn einen zeitlich ansteigenden Strom vorzugeben, wie auch im weiteren Fortschritt der Betriebsphase I einen konstanten Strom. Dadurch tritt eine Gewebeerwärmung und eine Elektrodenerwärmung auf. Durch thermische Gewebedenaturierung kommt es zu einer Verminderung der Gewebeimpedanz, die zum Beispiel zwischen 2 Ohm und 40 Ohm liegen kann. Durch Dampfbildung und beginnende Austrocknung des Gewebes kann die Impedanz während der Betriebsphase I wieder ansteigen, bis das Ende der Phase I erkannt wird. Dazu können verschiedene Erkennungskriterien genutzt werden. Zum Beispiel kann das Verhältnis zwischen Spannung und Strom an der Quelle und somit die Gewebeimpedanz über einen Grenzwert hinaus ansteigen. Alternativ kann als Erkennungskriterium genutzt werden, wenn das Verhältnis zwischen Spannung und Strom an der Quelle, d.h. die Gewebeimpedanz, ein Minimum durchläuft. Weiter alternativ kann als Erkennungskriterium genutzt werden, dass die Spannung an der Quelle einen Grenzwert übersteigt. Weiter alternativ kann als Erkennungskriterium genutzt werden, dass der von der Quelle konstant zu haltende Strom unter einen Schwellwert abfällt, weil beispielsweise die von der Steuereinheit und der Quelle gebildete Stromregelschaltung ihren Regelbereich verlässt. Dies kann geschehen, wenn die Quelle ihre Maximalspannung oder eine sonstige Spannungsgrenze erreicht hat. Auch kann alternativ die Geschwindigkeit der Änderung des Gewebewiderstands (Verhältnis zwischen Spannung und Strom an der Quelle) als Abschaltkriterium genutzt werden, zum Beispiel indem für die Anstiegsgeschwindigkeit der Gewebeimpedanz eine Grenze festgelegt und deren Erreichen überwacht wird.

Jedenfalls wird am Ende der Betriebsphase I die bislang applizierte Energie gespeichert. Der Fortgang der weiteren Steuerung der Betriebsphase II wird aus diesem Energiewert abgeleitet. Insbesondere kann die Dauer der Betriebsphase II entsprechend dem Energiewert aus der Betriebsphase I festgelegt werden. Auch kann das Abschaltkriterium, d.h. das Ende einer sich anschließenden Betriebsphase III, anhand des in der ersten Betriebsphase ermittelten Energiewerts festgelegt werden. Somit sind die Steuerungsparameter, d.h. die Dauer der Betriebsphase II und das Abschaltkriterium, d.h. das Ende der Betriebsphase III, Funktionen der in der Betriebsphase 1 gemessenen Energie. Vorzugsweise erfolgt der Übergang von der Betriebsphase I zur Betriebsphase II stetig, d.h. ohne sprungartige Änderung des an das biologische Gewebe gelieferten Stroms und/oder ohne sprungartige Änderung der an das Gewebe angelegten Spannung und/oder ohne sprungartige Änderung der an das Gewebe abgegebenen Leistung.

In der Betriebsphase II betreibt die Steuereinheit die Quelle vorzugsweise impedanzgeregelt als Sollwert des Impedanzanstiegs. Für die Gewebeimpedanz empfiehlt sich ein Wert oberhalb 100 Ohm pro Sekunde. Die gezielte langsame Erhöhung der Impedanz bewirkt eine Verstetigung der Verdampfung von Gewebeflüssigkeit. Die Dampfbildung erfolgt gleichmäßig und räumlich verteilt. Der gewünschte zeitliche Verlauf der Impedanz kann einen konstanten Anstieg oder auch einen variablen Anstieg haben. Vorzugsweise legt die Steuereinheit die zeitliche Länge der Betriebsphase II in Abhängigkeit von der in der ersten Betriebsphase erfassten Energie fest. Die zweite Betriebsphase wird beendet, wenn die Zeit $t_2$ verstrichen ist. Es schließt sich (optional) die dritte Betriebsphase III an. In dieser wird vorzugsweise an das biologische Gewebe eine konstante Spannung appliziert.

Das Ende der dritten Betriebsphase III kann dadurch festgelegt sein, dass die minimale Behandlungszeit verstrichen ist und eine Energie $E_{ges}$ erreicht worden ist. Die Energie $E_{ges}$ kann in Abhängigkeit von der in der ersten Betriebsphase erfassten Energie $E_1$ festgelegt sein. Die minimale Behandlungszeit $t_{min}$ kann ebenfalls von der Energie $E_1$ bestimmt werden. Alternativ kann die Betriebsphase III beendet werden, wenn die maximale Behandlungszeit verstrichen ist. Diese kann wiederum in Abhängigkeit von der minimalen Behandlungszeit und somit ebenfalls in Abhängigkeit von der in der ersten Betriebsphase erfassten Energie $E_1$ festgelegt werden. Weitere Abschaltkriterien in die jeweils in Abhängigkeit von der Energie $E_1$ stehen, können festgelegt werden.

Im Laufe der Behandlung kann es vorkommen, dass sich Behandlungsparameter ändern. Beispielsweise kann durch versehentliches zeitweiliges Lockern der Elektroden von dem biologischen Gewebe (Öffnen von Fusionsklemmen) nachdringende Gewebeflüssigkeit, wie Blut oder Spülflüssigkeit, den Prozess beeinflussen. Somit kann es notwendig werden, dass eine größere Energiemenge und längere Applikationszeit notwendig wird als ursprünglich aus der Energie $E_1$ abgeleitet wurde. Um in solchen Fällen eine ordnungsgemäße Fusion zu erreichen, kann während der zweiten (und/oder dritten) Betriebsphase die aktuelle Leistung beobachtet werden. Sofern die Leistung innerhalb eines Beobachtungszeitintervalls ein vorgegebenes Fenster aus minimaler Leistung $P_{min}$ und maximaler Leistung $P_{max}$ für einen nicht vernachlässigbar kurzen Zeitraum verlässt, kann die Applikationszeit, d.h. die Zeiten $t_2$ und $t_3$, sowie Rechenparameter $t_{min}$ und/oder $t_{max}$ entsprechend verlängert werden.

Weitere Einzelheiten von Ausführungsformen der Erfindung ergeben sich aus Ansprüchen, aus der Zeichnung und/ oder aus der nachfolgenden Beschreibung eines veranschaulichenden Beispiels. Es zeigen:

Figur 1 die erfindungsgemäße Einrichtung, in schematischer Darstellung.

Figur 2 eine Steuereinheit für die Einrichtung nach Figur 1, in ausschnittsweiser schematisierter Blockdarstellung und

Figur 3 Zeitdiagramme zur Erläuterung der Funktion der Steuereinheit.

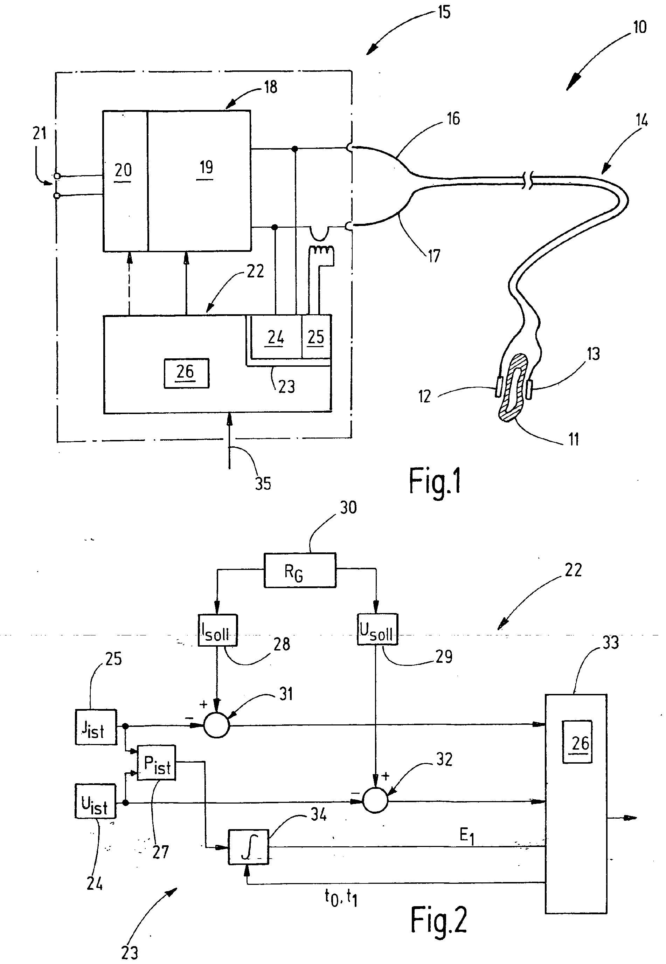

In Figur 1 ist eine Einrichtung 10 zur Koagulation von biologischem Gewebe 11 veranschaulicht, das beispielsweise ein Hohlgefäß oder auch irgendein anderes biologisches Gewebe sein kann. Im folgenden Beispiel ist als Gewebe 11 ein Blutgefäß veranschaulicht, das durch Koagulation geschlossen werden soll, d.h. es ist eine Fusion der einander gegenüber liegenden Wände des Gefäßes durchzuführen. Dazu dienen zwei Elektroden 12, 13, die zwischen einander das Gewebe 11 fassen und es auch mechanisch beanspruchen, beispielsweise zusammendrücken können. In Figur 1 ist die mechanische Struktur des entsprechenden Instruments nicht weiter veranschaulicht. Beispielsweise können die Elektroden 12, 13 die Branchen eines bipolaren Fusionsinstruments sein.

Die Elektroden 12, 13 sind über eine Leitung 14 mit einem speisenden Gerät 15 verbunden. Die Leitung 14 weist dazu zum Beispiel zwei Adern 16, 17 auf, denen von dem Gerät 15 hochfrequenter Strom zugeführt wird oder zugeführt werden kann.

Dazu weist das Gerät 15 eine Quelle 18, zum Beispiel in Gestalt eines steuerbaren HF-Generators 19, auf. Dieser kann über ein Netzteil 20 und einen Netzanschluss 21 über ein Versorgungsnetz mit Betriebsspannung versorgt sein.

Der HF-Generator 19 und/oder das Netzteil 20 sind steuerbar ausgebildet. An ihren entsprechenden Steuereingänge ist, wie Pfeile veranschaulichen, eine Steuereinheit 22 angeschlossen, die insbesondere die Abgabe elektrischer Leistung durch den HF-Generator 19 steuert oder regelt. Dazu enthält die Steuereinheit 22 eine Überwachungseinheit 23, die die elektrischen Größen der den Elektroden 12, 13 zugeführten Elektroenergie erfasst. Insbesondere ist die Überwachungseinheit 23 dazu eingerichtet, zumindest zeitweilig die den Elektroden 12, 13 zugeführte elektrische Leistung zu erfassen und aufzuintegrieren, um die in einem Zeitintervall gelieferte Energie zu bestimmen. Die Überwachungseinheit 23 kann einen Spannungsblock 24 zur Überwachung der an den Klemmen 12, 13 liegenden Spannung aufweisen. Außerdem kann die Überwachungseinheit 23 einen Stromblock 25 zur Bestimmung der Größe des zu den Elektroden 12, 13 gelieferten Stroms aufweisen. Die Steuereinheit 22 kann außerdem ein Modul 26 zur Festlegung des Endes einer ersten Betriebsphase I aufweisen, wobei das Modul wenigstens ein Ausgangssignal des Spannungsblocks 24 oder des Stromblocks 25 oder ein aus deren Ausgangssignalen abgeleitetes Signal zur Betriebsphasenenderkennung erhält.

Die Steuereinheit 22 ist in Figur 2 schematisch vereinfacht und lediglich auszugsweise veranschaulicht. Mit dem Stromblock 25 wird der durch das Gewebe 11 fließende Strom $I_{IST}$ erfasst. Mit dem Spannungsblock 24 wird die an das Gewebe 11 angelegte tatsächliche Spannung $U_{IST}$ erfasst. Aus beiden Größen wird, zumindest zeitweilig, die tatsächliche dem Gewebe 11 zugeführte Leistung $P_{IST}$ errechnet. Die Leistung $P_{IST}$ kann die erfasste Wirkleistung oder auch die den Elektroden 12, 13 zugeführte Scheinleistung sein. Zur Errechnung oder anderweitigen Bestimmung der Leistung $P_{IST}$ dient ein entsprechender Block 27.

Die Steuereinheit 22 kann außerdem einen Stromvorgabeblock 28 aufweisen, der zeit- und/oder situationsabhängig einen Strom $I_{SOLL}$ vorgibt. Ebenso kann ein Spannungsvorgabeblock 29 vorgesehen sein, um eine gewünschte Spannung $U_{SOLL}$ vorzugeben. Der Stromvorgabeblock 28 und der Spannungsvorgabeblock 29 können von einem Impedanzblock 30 gesteuert sein, der ein gewünschtes Verhältnis zwischen der Spannung $U_{SOLL}$ und dem Strom $I_{SOLL}$ zeit- und oder situationsabhängig festlegt, beispielsweise um einen gewünschten Gewebewiderstand $R_G$ oder einen gewünschten zeitlichen Verlauf desselben festzulegen.

In entsprechenden Differenzbildungsblöcken 31, 32 werden jeweils die Soll-Ist-Abweichungen für den Strom $I_{IST}$ und die Spannung $U_{IST}$ gebildet und einem Verarbeitungsmodul 33 zugeführt. Letzteres steuert den Generator 19.

Das Verarbeitungsmodul 33 enthält außerdem das Modul 26 zur Erkennung verschiedener Betriebsphasen. Dieses Modul 26 kann (über nicht veranschaulichte Signalwege) wenigstens den tatsächlichen Strom $I_{IST}$ und/oder die tatsächliche $U_{IST}$ Spannung oder einen aus diesen Größen abgeleiteten Wert als Eingangsgröße erhalten.

An den Block 27 zur Leistungsermittlung ist ein Energieblock 34 zur Bestimmung der an das Gewebe 11 gelieferten Energie angeschlossen. Dieser integriert die gemessene Leistung $P_{IST}$ über einen von dem Verarbeitungsmodul 33 vorgegebenen Zeitraum auf und liefert das Integral an den Verarbeitungsblock 33.

Es wird darauf hingewiesen, dass die Blöcke 27 bis 32 sowie 34 auch Teil des Verarbeitungsmoduls 33 sein können.

Der weitere Aufbau des Geräts 15 und insbesondere seiner Steuereinheit 22 ergibt sich aus der nachfolgenden Beschreibung von deren Zeitverhalten:

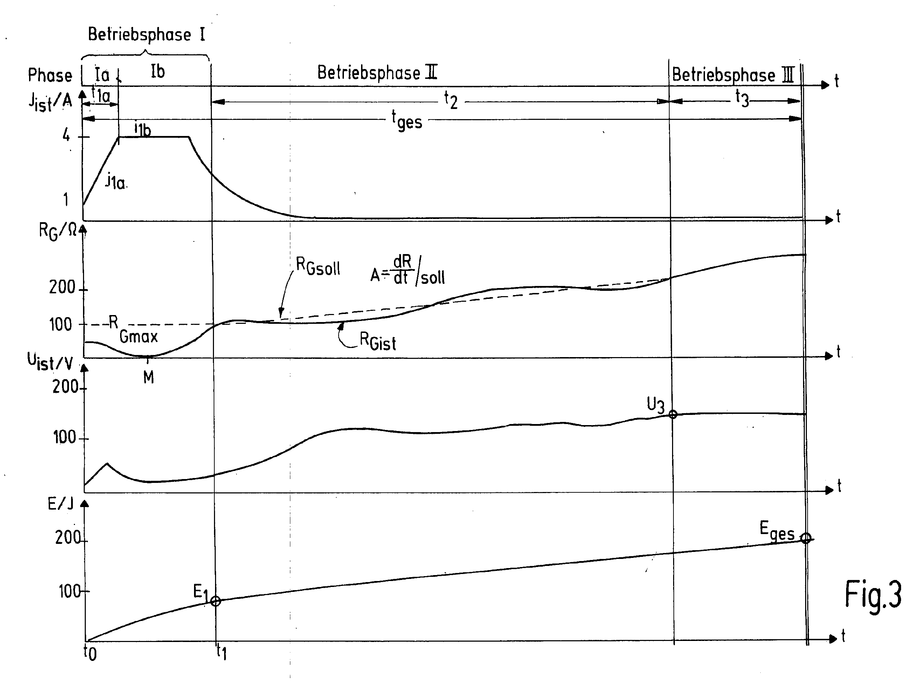

Es wird davon ausgegangen, dass zunächst zwischen den Elektroden 12, 13 lebendes, nicht denaturiertes Gewebe 11 gefasst ist. Das Gerät 15 erhält nun an seinem Aktivierungseingang 35 das Signal zur Koagulation und gegebenenfalls Fusion des biologischen Gewebes 11. Dies entspricht dem in Figur 3 vermerkten Anfangszeitpunkt bzw. Aktivierungsbeginn $t_0$. Es beginnt die Betriebsphase I zunächst mit einer Teilphase Ia. In dieser wird der Strom $I_{IST}$ kontrolliert auf einen gewünschten Stromwert von zum Beispiel 4 A gebracht. Er kann dabei von einem Ausgangswert, wie beispielsweise 1 A innerhalb einer Zeitspanne $t_{1a}$ auf den Sollwert von zum Beispiel 4 A geführt werden. Dies kann in einer linearen Rampe geschehen: Die Zeit dafür kann zwischen 200 ms und 2 s betragen. Vorzugsweise wird als Messgröße der Effektivwert des Stroms genommen. Während dieser Phase oder auch ganz oder teilweise in einer späteren Betriebsphase Ib fällt der Gewebewiderstand $R_G$ von einem initialen Wert auf einen minimalen Wert von zum Beispiel 2 Ohm bis 40 Ohm ab. Durch das Erhöhen des Stroms steigt die Spannung $U_{IST}$ während der Zeitdauer $t_{1a}$ an. Der Strom $I_{ist}$ wird während dieser Zeit vorzugsweise in Form einer Rampe erhöht. Als Messwert für die Spannung $U_{IST}$ kann zum Beispiel die Spitzenspannung zwischen den Elektroden 12, 13 gemessen werden. In der Betriebsphase I wird dann während einer weiteren Teilphase Ib der Strom $I_{IST}$ auf den Wert $i_{1b}$ konstant gehalten. Dabei arbeitet die Steuereinheit 22 als Stromregelschaltung zur Konstanthaltung des Werts $i_{1b}$.

Während der ersten Teilphase Ia oder während der zweiten Teilphase Ib durchläuft der Gewebewiderstand $R_G$ ein Minimum, um dann wieder anzusteigen. Wird das Gewebewiderstands-Minimum schon in der ersten Teilphase Ia erreicht, kann die Teilphase Ib übersprungen und direkt in die Betriebsphase II übergegangen werden. Dabei kann unter Umständen die Leistungsgrenze des Generators 19 erreicht werden, so dass es der Stromregelschaltung nicht mehr gelingt, den Strom $I_{IST}$ mit dem gewünschten Strom $I_{SOLL}$ in Übereinstimmung zu bringen. Der Strom fällt somit gegen Ende der Betriebsphase I ab. Je nach Ausführungsform kann dieser Abfall des Stroms $i_{1b}$ oder auch der von dem Differenzbildungsblock 31 gebildete Stromdifferenzwert ($I_{SOLL}$ - $I_{IST}$) als Kennzeichen für das Ende der Betriebsphase I genutzt werden. Es ist auch möglich, dass die Steuereinheit 22 die Gewebeimpedanz $R_G$ als Quotient aus $U_{IST}$ und $I_{IST}$ ermittelt und das Ende der Betriebsphase I feststellt, wenn der Gewebewiderstand eine gegebene Schwelle überschreitet. Alternativ kann auch die Anstiegsgeschwindigkeit für den Gewebewiderstand $R_G$ überwacht werden. Demnach können kumulativ oder alternativ von der Steuereinheit 22 folgende Kriterien zur Erkennung der Betriebsphase I genutzt werden:

- Detektion des Durchlaufens des Minimums der Gewebeimpedanz oder des Gewebewiderstands dR/dt = 0)
- Unterschreiten eines Schwellwerts des Stroms $I_{IST}$, zum Beispiel 0,5 * $i_{1b}$
- Überschreiten eines Schwellwerts der Gewebeimpedanz, zum Beispiel von 80 Ohm
- Überschreiten eines Schwellwerts der Anstiegsgeschwindigkeit der Gewebeimpedanz (dR/dt)

Während der gesamten Betriebsphase I integriert der Energieblock 34 die von dem Block 27 ermittelte Leistung und liefert am Ende der Betriebsphase I den ermittelten Wert der Energie $E_1$ an das Verarbeitungsmodul 33. Anfang und Ende der Betriebsphase I werden durch die Zeitpunkte $t_0$ und $t_1$ markiert. Der Zeitpunkt $t_1$ wird von dem Verarbeitungsmodul 33 nach einem der oben genannten Kriterien bestimmt.

Mit dem Ende der Betriebsphase I beginnt die Betriebsphase II. Diese beginnt vorzugsweise mit dem gleichen Strom $I_{IST}$, mit der die Betriebsphase I geendet hat. Außerdem beginnt sie vorzugsweise mit der gleichen Spannung $U_{IST}$, mit der die erste Betriebsphase I geendet hat. Für die Betriebsphase II werden nun Betriebskriterien anhand der in der Betriebsphase I ermittelten applizierten Energie $E_1$ festgelegt. Vorzugsweise wird der Generator 19 in der Betriebsphase II impedanzgeregelt betrieben, d.h. die Steuereinheit 22 bildet einen Regler für die Gewebeimpedanz. Für die Gewebeimpedanz wird ein gewünschter zeitlicher Impedanzanstieg A festgelegt. In Figur 3 ist der Impedanzanstieg A als gewünschte gestrichelte Linie im Zeitverlauf als $R_{Gsoll}$ veranschaulicht. Der tatsächliche Impedanzanstig $R_{Gist}$ kann davon etwas abweichen, dies hängt von der Regelgüte des von der Steuereinheit 22 nun gebildeten Impedanzreglers ab. Zugleich fällt während der Betriebsphase II, d.h. während der Zeitdauer $t_2$ der Strom $I_{IST}$ ab, während die Spannung $U_{IST}$ ansteigt. Die Spannung $U_{ist}$ hat eine obere Grenze, z.B. 150V (Scheitelwert), so dass vermieden wird, dass Funken auftreten und somit eine Schnittwirkung hervorgerufen würde.

Der Impedanzanstig A kann 50 bis 200, vorzugsweise 100 Ohm pro Sekunde betragen. Die gezielte langsame Erhöhung der Impedanz bewirkt eine Verstetigung der Verdampfung der Gewebeflüssigkeit.

Die Betriebsphase II wird beendet, wenn die Dauer $t_2$ verstrichen ist. Die Dauer $t_2$ kann auf folgende Weise aus der Energie $E_1$ ermittelt werden:

$$t_2 = 2/3 \ (t_{max} - t_1),$$

Die Zeit $t_{max}$ ist dabei die maximale Behandlungsdauer. Die maximale Behandlungsdauer $t_{max}$ lässt sich aus der minimalen Behandlungsdauer errechnen, indem ein konstanter vorgegebener Summand addiert wird, zum Beispiel:

$$t_{max} = t_{min} + 1,8s$$

Die minimale Behandlungsdauer $t_{min}$ lässt sich beispielsweise nach folgender Beziehung aus der Energie $E_1$ bestimmen:

$$t_{min} = min \ \{5,4s; (-38,25\mu s * E_1^2 / J^2 + 18ms * E_1 / J + 270ms)\}.$$

Danach ist $t_{min}$ ein festgelegter Wert von zum Beispiel 5,4 s oder der sich durch Ausrechnen der runden Klammer ergebende Wert, je nachdem, welcher Wert geringer ist.

Mit Ende der Betriebsphase II wird die Betriebsphase III begonnen. In dieser wird die Spannung $U_{IST}$ auf den Wert $U_3$ für eine Zeitspanne $t_3$ konstant geregelt. Die Steuereinheit 22 arbeitet hier als Spannungsreglerschaltung.

Während der Betriebsphasen II und III, die der Phase II der Gewebekoagulation entsprechen, wird die Leistung weiter aufintegriert. Erreicht dieser Wert den Gesamthöchstwert $E_{ges}$, wird die Behandlung beendet. Der Gesamthöchstwert $E_{ges}$ kann nach verschiedenen empirisch gewonnenen Formeln in Abhängigkeit von der Energie $E_1$ ermittelt werden, beispielsweise folgendermaßen:

$$E_{ges} = 45J + 2,75 * E_1.$$

Alternativ kann das Erreichen der maximalen Dauer $t_3$ der Betriebsphase III erkannt werden. Diese Dauer $t_3$ kann zum Beispiel nach:

$$t_3 = 1/3 * (t_{max} - t_1)$$

berechnet werden.

Zur Vermeidung von Fehlbehandlungen durch unvorhergesehene Änderungen der Behandlungsparameter, beispielsweise durch versehentliches Öffnen der Fusionsklemmen, kann zusätzlich überwacht werden, ob innerhalb eines Beobachtungszeitintervalls, beispielsweise während der Betriebshase II und/oder III, die tatsächliche Leistung ein Leistungsfenster aus $P_{min}$ und $P_{max}$ verlässt. Falls dies für längere Zeit der Fall ist, kann die Applikationszeit verlängert werden.

Eine Einrichtung 10 zur Gewebekoagulation, insbesondere Fusion, weist eine elektrische Quelle 18 auf, die mit Elektroden 12, 13 zur Stromeinwirkung auf biologisches Gewebe 11 verbunden oder verbindbar ist. Eine Steuereinheit 22 steuert die Quelle 18 während der Phasen I und II der Gewebefusion. Diesen Phasen I und II entsprechen Betriebsphasen I, II und III der Einrichtung 10. Während der Betriebsphase I erfasst eine Überwachungseinheit 23, die in das Gewebe 11 applizierte Energie $E_1$. Die Steuereinheit 22 steuert die Quelle 18 in den nachfolgenden Betriebsphasen II und III anhand der erfassten Energie $E_1$. Eine solche Einrichtung erweist sich als besonders zuverlässig und im Einsatz robust Die Erfindung ist in den folgenden Ansprüchen definiert.

Bezugszeichenliste:

| | |
|---|---|
| 10 | Einrichtung |
| 11 | biologisches Gewebe |
| 12, 13 | Elektroden |
| 14 | Leitung |
| 15 | Gerät |
| 16, 17 | Adern |
| 18 | Quelle |
| 19 | HF-Generator |
| 20 | Netzteil |
| 21 | Netzanschluss |
| 22 | Steuereinheit |
| 23 | Überwachungseinheit |
| 24 | Spannungsblock |
| 25 | Stromblock |
| 26 | Modul zur Erkennung von Betriebsphasen |
| $U_{IST}$ | Spannung (z.B. Spitzenwert) |
| $I_{IST}$ | Strom (z.B. Effektivwert) |
| $P_{IST}$ | Leistung |
| 27 | Block zur Leistungsermittlung |
| 28 | Stromvorgabeblock |
| $I_{SOLL}$ | gewünschter Strom |
| 29 | Spannungsvorgabeblock |
| $U_{SOLL}$ | gewünschte Spannung |
| 30 | Impedanzblock |
| $R_G$ | Gewebewiderstand |
| 31, 32 | Differenzbildungsblöcke |
| 33 | Verarbeitungsmodul |
| 34 | Energieblock |
| 35 | Aktivierungseingang |
| $t_0$ | Aktivierungsbeginn |
| I | erste Betriebsphase |
| Ia | Teilphase |
| $t_{1a}$ | Dauer der ersten Teilphase |
| Ib | Teilphase |
| $i_{1a}$ | Wert des Stroms $I_{IST}$ in der Teilphase Ia |
| $i_{1b}$ | Wert des Stroms $I_{IST}$ in der Teilphase Ib |
| $t_1$ | Dauer der Betriebsphase I |
| $E_1$ | in Phase I in das Gewebe 11 eingetragene Energie |
| A | Impedanzanstieg |
| $R_{Gsoll}$ | gewünschter Impedanzverlauf |
| $R_{Gist}$ | tatsächlicher Impedanzverlauf |
| $t_2$ | Dauer der Betriebsphase II |
| $t_{max}$ | maximale Behandlungsdauer |
| $t_{min}$ | minimale Behandlungsdauer |
| $E_{ges}$ | Gesamthöchstwert der Energie |
| $t_3$ | Dauer der Betriebsphase III |
| $t_{ges}$ | gesamte Dauer der Behandlung |
| $R_{Gmax}$ | Grenzwert für Gewebewiderstand in der Betriebsphase I |
| M | Minimum des Gewebewiderstands in der Betriebsphase I |
| $U_3$ | Spannung in der Betriebsphase III |
| $P_{max}$, $P_{min}$ | legen Leistungsfenster für die Leistung P der Quelle 18 in den Betriebsphasen II und/oder III fest |

**Patentansprüche**

1. Einrichtung (10) zur Gewebekoagulation, insbesondere zur Gewebefusion,
   mit einer elektrischen Quelle (18), die mit Elektroden (12, 13) zur Stromeinwirkung auf biologisches Gewebe (11) verbunden ist,
   mit einer Überwachungseinheit (23), die an die Quelle (18) angeschlossen ist, um den von der Quelle (18) abgegebenen Strom ($I_{IST}$) und/oder die von der Quelle (18) abgegebene Spannung ($U_{IST}$) zu erfassen,
   mit einer Steuereinheit (22), die die Überwachungseinheit (23) enthält und mit der Quelle (18) steuernd verbunden ist, wobei die Steuereinheit(22) in einer ersten Betriebsphase (I) die von der Quelle (18) an die Elektroden (12, 13) abgegebene Energie ($E_1$) ermittelt und speichert und in einer darauf folgenden zweiten Betriebsphase (II) die Quelle (18) in Abhängigkeit von der in der ersten Betriebsphase (I) erfassten Energie ($E_1$) steuert, wobei die zeitliche Länge (t2) der zweiten Betriebsphase (II) von der Steuereinheit (22) in Abhängigkeit von der in der ersten Betriebsphase (I) erfassten Energie (E1) festgelegt wird.

2. Einrichtung nach Anspruch 1, **dadurch gekennzeichnet, dass** die Steuereinheit (22) mit der Quelle (18) in der ersten Betriebsphase (I) als Stromregelschaltung zusammengeschaltet ist.

3. Einrichtung nach einem der vorstehenden Ansprüche, **dadurch gekennzeichnet, dass** die Steuereinheit (22) zu Beginn der ersten Betriebsphase (I) einen zeitlich ansteigenden Strom ($i_{1a}$) vorgebend ausgebildet ist.

4. Einrichtung nach einem der vorstehenden Ansprüche, **dadurch gekennzeichnet, dass** die Steuereinheit (22) während zumindest eines Abschnitts (Ib) der ersten Betriebsphase (I) einen konstanten Strom ($i_{1b}$) vorgebend ausgebildet ist.

5. Einrichtung nach einem der vorstehenden Ansprüche, **dadurch gekennzeichnet, dass** die Steuereinheit (22) ein Modul (26) zur Erfassung des Abschlusses der ersten Betriebsphase (I) aufweist, wobei das Modul (26) dazu ausgebildet ist, wenigstens eines der folgenden Erkennungskriterien zu nutzen:

   - das Verhältnis zwischen Spannung und Strom an der Quelle (18) steigt über einen Grenzwert ($R_{Gmax}$) an,
   - das Verhältnis zwischen Spannung und Strom an der Quelle (18) durchläuft ein Minimum (M),
   - die Anstiegsgeschwindigkeit des Verhältnisses zwischen Spannung und Strom an der Quelle (18) übersteigt einen Grenzwert,
   - die Spannung ($U_{IST}$) an der Quelle (18) übersteigt einen Grenzwert,
   - der Strom ($I_{IST}$) unterschreitet einen Grenzwert.

6. Einrichtung nach einem der vorstehenden Ansprüche, **dadurch gekennzeichnet, dass** die Steuereinheit (22) darauf eingerichtet ist, zu Beginn der zweiten Betriebsphase (II) wenigstens eine der folgenden Größen:

   - Strom ($I_{IST}$) aus der Quelle (18),
   - Spannung ($U_{IST}$) an der Quelle (18),
   - abgegebene Leistung ($P_{IST}$) der Quelle (18)

   auf den gleichen Wert einzustellen, den diese Größe zu Ende der ersten Betriebsphase (I) hatte.

7. Einrichtung nach einem der vorstehenden Ansprüche, **dadurch gekennzeichnet, dass** die Steuereinheit (22) in der zweiten Betriebsphase (II) einen Zeitverlauf für das Verhältnis aus der Spannung ($U_{IST}$) an der Quelle (18) und dem von dieser gelieferten Strom ($I_{IST}$) vorgebend ausgebildet ist.

8. Einrichtung nach Anspruch 7, **dadurch gekennzeichnet, dass** der Zeitverlauf einen konstanten Impedanzanstieg (A) aufweist.

9. Einrichtung nach einem der vorstehenden Ansprüche, **dadurch gekennzeichnet, dass** die Steuereinheit (22) die zeitliche Länge ($t_2$) der zweiten Betriebsphase (II) vorgebend ausgebildet ist.

10. Einrichtung nach einem der vorstehenden Ansprüche, **dadurch gekennzeichnet, dass** die Steuereinheit (22) im unmittelbaren Anschluss an die zweite Betriebsphase (II) in eine dritte Betriebsphase (III) übergehend ausgebildet ist.

11. Einrichtung nach Anspruch 10, **dadurch gekennzeichnet, dass** die Steuereinheit (22) in der dritten Betriebsphase (III) eine konstante Spannung ($U_3$) vorgebend und einstellend ausgebildet ist.

12. Einrichtung nach einem der Ansprüche 10 oder 11 **dadurch gekennzeichnet, dass** die Steuereinheit (22) in der dritten Betriebsphase (III) die Spannung ($U_3$) der Quelle (18) auf denjenigen Wert festlegend ausgebildet ist, der zu Ende der zweiten Betriebsphase (II) durch die Überwachungseinheit (23) erfasst worden ist.

13. Einrichtung nach einem der Ansprüche 10 bis 12 **dadurch gekennzeichnet, dass** die Steuereinheit (22) die dritte Betriebsphase (III) beendend ausgebildet ist, wenn:

    - eine minimale Behandlungszeit ($t_{min}$) und eine gegebene Gesamtenergie ($E_{ges}$) erreicht sind oder
    - eine maximale Behandlungszeit ($t_{max}$) verstrichen ist oder
    - eine maximale Energie ($E_{max}$) appliziert worden ist.

14. Einrichtung nach einem der Ansprüche 10 bis 12 **dadurch gekennzeichnet, dass** die Steuereinheit (22) dazu eingerichtet ist, in der Betriebsphase (II) die von der Quelle (18) abgegebene Leistung zu überwachen, um die Zeitdauer ($t_2$) für die zweite oder dritte Betriebsphase (II, III) zu verlängern, sofern die Leistung ein zwischen einer Maximalleistung ($P_{max}$) und einer Minimalleistung ($P_{min}$) vorgegebenes Leistungsfenster verlassen hat.

## Claims

1. Device (10) for tissue coagulation, in particular for tissue fusion,
with an electrical source (18) which is connected to electrodes (12, 13) for the action of current on biological tissue (11),
with a monitoring unit (23) which is connected to the source (18) for detecting the current ($I_{IST}$) emitted by the source (18) and/or the voltage ($U_{IST}$) emitted by the source (18),
with a control unit (22) which contains the monitoring unit (23) and is connected for control purposes to the source (18), wherein the control unit (22)

    - in a first operating phase (I), determines and stores the energy ($E_1$) output by the source (18) to the electrodes (12, 13), and
    - in a subsequent second operating phase (II), controls the source (18) depending on the energy ($E_1$) detected in the first operating phase (I), wherein the temporal length (t2) of the second operating phase (II) is established by the control unit (22) depending on the energy ($E_1$) detected in the first operating phase (I).

2. Device according to claim 1, **characterised in that** in the first operating phase (I), the control unit (22) is connected together with the source (18) as a current regulation circuit.

3. Device according to one of the preceding claims, **characterised in that** the control unit (22) is configured so as to predefine a temporally rising current ($i_{1a}$) at the start of the first operating phase (I).

4. Device according to any of the preceding claims, **characterised in that** the control unit (22) is configured so as to predefine a constant current ($i_{1b}$) during at least a portion (Ib) of the first operating phase (I).

5. Device according to any of the preceding claims, **characterised in that** the control unit (22) comprises a module (26) for detecting the end of the first operating phase (I), wherein the module (26) is configured to use at least one of the following detection criteria:

    - the ratio between voltage and current at the source (18) rises above the limit value ($R_{Gmax}$),
    - the ratio between voltage and current at the source (18) passes through a minimum (M),
    - the rise rate of the ratios between voltage and current at the source (18) exceeds a limit value,
    - the voltage ($U_{IST}$) at the source (18) exceeds a limit value,
    - the current ($I_{IST}$) falls below a limit value.

6. Device according to any of the preceding claims, **characterised in that** the control unit (22) is configured to set, at the start of the second operating phase (II), at least one of the following parameters:

    - current ($I_{IST}$) at the source
    - voltage ($U_{IST}$) at the source

- output power ($P_{IST}$) at the source (18)

to the same value as this parameter had at the end of the first operating phase (I).

7. Device according to any of the preceding claims, **characterised in that** the control unit (22) is configured so as to predefine, in the second operating phase (II), a time curve for the ratio between the voltage ($U_{IST}$) at the source (18) and the current ($I_{IST}$) supplied thereby.

8. Device according to claim 7, **characterised in that** the time curve has a constant impedance rise (A).

9. Device according to any of the preceding claims, **characterised in that** the control unit (22) is configured so as to predefine the temporal length ($t_2$) of the second operating phase (II).

10. Device according to any of the preceding claims, **characterised in that** the control unit (22) is configured so as to transfer into a third operating phase (III) immediately following the second operating phase (II).

11. Device according to claim 10, **characterised in that** the control unit (22) is configured so as to predefine and set a constant voltage ($U_3$) in the third operating phase (III).

12. Device according to one of claims 10 or 11, **characterised in that**, the control unit (22) is configured so as to set, in the third operating phase (III), the voltage ($U_3$) from the source (18) to the value which was detected by the monitoring unit (23) at the end of the second operating phase (II).

13. Device according to any of claims 10 to 12, **characterised in that** the control unit (22) is configured so as to end the third operating phase (III) when:

- a minimal treatment time ($t_{min}$) and a given total energy ($E_{ges}$) have been reached, or
- a maximal treatment time ($t_{max}$) has elapsed, or
- a maximal energy ($E_{max}$) has been applied.

14. Device according to any of claims 10 to 12, **characterised in that** the control unit (22) is configured so as to monitor, in the operating phase (II), the power output by the source (18) in order to extend the duration ($t_2$) for the second or third operating phase (II, III) if the power has strayed beyond a predefined power window between a maximal power ($P_{max}$) and a minimal power (Pmin).

**Revendications**

1. Dispositif (10) destiné à la coagulation de tissus, notamment à la fusion tissulaire,
comprenant une source électrique (18) qui est reliée à des électrodes (12, 13) en vue de l'action du courant sur des tissus biologiques (11),
comprenant une unité de surveillance (23) qui est raccordée à la source (18) afin de mesurer le courant ($I_{ist}$) délivré par la source (18) et/ou la tension ($U_{ist}$) délivrée par la source (18),
comprenant une unité de commande (22) qui contient l'unité de surveillance (23) et est reliée à la source (18) de manière à la commander, sachant que l'unité de commande (22),
au cours d'une première phase de fonctionnement (I), détermine et enregistre l'énergie ($E_1$) fournie par la source (18) aux électrodes (12, 13), et
au cours d'une deuxième phase de fonctionnement (II) qui suit, commande la source (18) en fonction de l'énergie ($E_1$) mesurée lors de la première phase de fonctionnement (I), sachant
que la longueur dans le temps (t2) de la deuxième phase de fonctionnement (II) est fixée par l'unité de commande (22) en fonction de l'énergie ($E_1$) mesurée lors de la première phase de fonctionnement (I).

2. Dispositif selon la revendication 1, **caractérisé en ce que**, pendant la première phase de fonctionnement (I), l'unité de commande (22) est connectée à la source (18) pour former un circuit de régulation de courant.

3. Dispositif selon l'une des revendications précédentes, **caractérisé en ce que** l'unité de commande (22) est conçue pour prédéterminer un courant ($i_{1a}$) qui augmente dans le temps au début de la première phase de fonctionnement (I).

**EP 2 805 682 B1**

**4.** Dispositif selon l'une des revendications précédentes, **caractérisé en ce que** l'unité de commande (22) est conçue pour prédéterminer un courant ($i_{1b}$) constant, au moins pendant une partie (Ib) de la première phase de fonctionnement (I).

**5.** Dispositif selon l'une des revendications précédentes, **caractérisé en ce que** l'unité de commande (22) présente un module (26) destiné à détecter la fin de la première phase de fonctionnement (I), le module (26) étant conçu pour utiliser au moins l'un des critères de détection suivants :

- le rapport entre la tension et le courant sur la source (18) augmente au-delà d'une valeur limite ($R_{Gmax}$),
- le rapport entre la tension et le courant sur la source (18) passe par un minimum (M),
- la vitesse d'augmentation du rapport entre la tension et le courant sur la source (18) dépasse une valeur limite,
- la tension ($U_{IST}$) à la source (18) dépasse une valeur limite,
- le courant ($I_{IST}$) passe en dessous d'une valeur limite.

**6.** Dispositif selon l'une des revendications précédentes, **caractérisé en ce que** l'unité de commande (22) est conçue pour régler, au début de la deuxième phase de fonctionnement (II), au moins l'une des grandeurs suivantes :

- le courant ($I_{IST}$) provenant de la source (18),
- la tension ($U_{IST}$) sur la source (18),
- la puissance ($P_{IST}$) délivrée par la source (18),

pour qu'ils adoptent la même valeur que celle qu'avait cette grandeur à la fin de la première phase de fonctionnement (I).

**7.** Dispositif selon l'une des revendications précédentes, **caractérisé en ce que** l'unité de commande (22) est conçue pour prédéterminer, au cours de la deuxième phase de fonctionnement (II), une évolution dans le temps pour le rapport entre la tension ($U_{IST}$) à la source (18) et le courant ($I_{IST}$) fourni par celle-ci.

**8.** Dispositif selon la revendication 7, **caractérisé en ce que** l'évolution dans le temps présente une augmentation d'impédance (A) constante.

**9.** Dispositif selon l'une des revendications précédentes, **caractérisé en ce que** l'unité de commande (22) est conçue pour prédéterminer la longueur dans le temps ($t_2$) de la deuxième phase de fonctionnement (II).

**10.** Dispositif selon l'une des revendications précédentes, **caractérisé en ce que** l'unité de commande (22) est conçue pour passer à une troisième phase de fonctionnement (III), immédiatement à la suite de la deuxième phase de fonctionnement (II).

**11.** Dispositif selon la revendication 10, **caractérisé en ce que** l'unité de commande (22) est conçue pour prédéterminer et régler une tension ($U_3$) constante au cours de la troisième phase de fonctionnement (III).

**12.** Dispositif selon l'une des revendications 10 ou 11, **caractérisé en ce que** l'unité de commande (22) est conçue pour fixer, au cours de la troisième phase de fonctionnement (III), la tension ($U_3$) de la source (18) sur la valeur qui a été mesurée par l'unité de surveillance (23) à la fin de la deuxième phase de fonctionnement (II).

**13.** Dispositif selon l'une des revendications 10 à 12, **caractérisé en ce que** l'unité de commande (22) est conçue pour terminer la troisième phase de fonctionnement (III), lorsque :

- un temps de traitement minimal ($t_{min}$) et une énergie totale ($E_{ges}$) donnée sont atteints, ou
- un temps de traitement maximal ($t_{max}$) s'est écoulé, ou
- une énergie maximale ($E_{max}$) a été appliquée.

**14.** Dispositif selon l'une des revendications 10 à 12, **caractérisé en ce que** l'unité de commande (22) est conçue pour surveiller, au cours de la phase de fonctionnement (II), la puissance délivrée par la source (18), afin de prolonger la durée ($t_2$) pour la deuxième ou la troisième phase de fonctionnement (II, III), dans la mesure où la puissance a quitté une fenêtre de puissance prédéfinie entre une puissance maximale ($P_{max}$) et une puissance minimale (Pmin).

Fig.1

Fig.2

Fig.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1862137 A1
- US 8216223 B2
- US 8034049 B2
- EP 2213255 B1
- EP 2394593 A1
- US 6733498 B2
- US 8147485 B2
- US 20100179563 A1
- US 20110160725 A1
- US 2013006237 A1
- EP 2499982 A1
- WO 2008102154 A1